# EUROPÄISCHE PATENTSCHRIFT

(11) **EP 0 997 104 B1**
(45) Veröffentlichungstag und Bekanntmachung des Hinweises auf die Patenterteilung: **01.12.2004**
(21) Anmeldenummer: 98118533.3
(22) Anmeldetag: 30.09.1998
(51) Int. Cl.: A61B 5/103

(54) **Vorrichtung zur messtechnischen Erfassung von Parametern der Haut**
Device for measuring parameters of the skin
Dispositif de mesure des paramètres de la peau

(43) Veröffentlichungstag der Anmeldung: 03.05.2000
(73) Patentinhaber: COURAGE + KHAZAKA ELECTRONIC GmbH, D-50829 Köln (DE)
(72) Erfinder: Khazata, Gabriel, 50859 Köln (DE)
(74) Vertreter: Dallmeyer, Georg, Dipl.-Ing.

(56) Entgegenhaltungen:
- EP-A- 0 129 598
- EP-A- 0 312 736
- EP-A- 0 783 867
- WO-A-96/25884
- FR-A- 2 427 084

## Beschreibung

Die Erfindung betrifft eine Vorrichtung sowie ein Verfahren zur messtechnischen Erfassung von Parametern der Haut nach dem Oberbegriff des Anspruchs 1 bzw. nach Anspruch 14.

Es sind dermographische Geräte bekannt, die Hauttopographie, z.B. hinsichtlich Abschuppungen oder Falten, messtechnisch zu erfassen, wobei bestimmte Hautparameter vermessen werden können.

Beispielsweise ist es bekannt, Hautveränderungen mit Hilfe einer Fotokamera zu dokumentieren, die mit einer Makrooptik versehen ist.

Aus der EP-A-0312736 ist ein Analysegerät zur Messung der Hauttopographie bekannt, das eine Videokamera aufweist, die von einer ringförmigen Beleuchtungseinrichtung umgeben ist. Die Videokamera kann auf die Hauptoberfläche aufgesetzt werden.

Die Videokamera ist an einer Bildverarbeitungseinrichtung angeschlossen, die die Speicherung und Wiedergabe, sowie ein Vermessen und Analysieren der Abbildungen ermöglicht.

Aus der WO-A-9625884 ist eine Messeinrichtung zur Bestimmung von Fettausscheidungen der Haut bekannt. Dabei wird eine auf einem Träger angeordnete opake Folie nach dem Auflegen auf die Hautoberfläche durch die Aufnahme der Fettausscheidungen der Haut lichtdurchlässig. Nachteilig bei dieser Messeinrichtung ist, dass eine opto-elektrische Auswertung erst nach der Applikation möglich ist und dadurch eine kontinuierliche Bestimmung der Fettausscheidung nicht oder nur sehr aufwendig möglich ist.

Die bekannten Geräte zur meßtechnischen Erfassung der Hautoberfläche haben den Nachteil, keine ausreichend kontrastreichen Bilder zu liefern, so daß die Bildauswertung erschwert ist.

Der Erfindung liegt demzufolge die Aufgabe zugrunde, eine Vorrichtung der eingangs genannten Art derart weiterzubilden, daß eine statische und/oder dynamische Messung von Hautabsonderungen möglich ist. Diese Aufgabe wird durch die Vorrichtung gemäß Hauptanspruch 1 gelöst.

Die Erfindung sieht in vorteilhafter Weise vor, daß zur Messung von Absonderungen der Haut eine die Absonderungen absorbierende, zunächst undurchsichtige, mit der Aufnahme der Absonderungen transparent werdende Folie mit Abstand vor der Optik auswechselbar angeordnet ist. Eine derartige Vorrichtung ermöglicht eine dynamische Messung der Produktionsgeschwindigkeit der Hautabsonderungen, wobei mit Hilfe der Videokamera eine zeitliche Dokumentation des Absorptionsverlaufs in der Folie möglich ist.

Zur Messung von Sebumabsonderungen der Haut wird eine fettabsorbierende, zunächst undurchsichtige, mit der Fettaufnahme transparent werdende Folie verwendet, während zur Messung der Feuchtigkeitsabsonderung der Haut eine feuchtigkeitsabsorbierende, zunächst undurchsichtige mit der Aufnahme von Feuchtigkeit transparent werdende Folie verwendet wird.

Die Anordnung der lichtemittierenden Einrichtung innerhalb des auf die Hautoberfläche aufsetzbaren Applikators ermöglicht die kontrastreiche Aufnahme der Hautoberfläche, wobei die Lichtquelle entweder extern angeordnet ist und mit der lichtemittierenden Einrichtung über Lichtleiter verbunden ist oder intern in den Applikator integriert ist.

Die Verwendung eines Neon-Lichtrohrs als Lichtquelle ermöglicht es, eine relativ zum sichtbaren Licht kürzere Wellenlänge zu verwenden, die zu einer Kontrasterhöhung führt.

Vorzugsweise ist die lichtemittierende Einrichtung ringförmig um die Optik der Videokamera angeordnet. Dadurch erfolgt eine gleichmäßige, schattenfreie Ausleuchtung der Hautoberfläche.

Das Videosignal der Videokamera kann per Funk an die Bildverarbeitungseinrichtung übertragbar sein. In diesem Fall ist der Applikator mit Videokamera frei beweglich und von einem Kabel unabhängig.

Die Lichtquelle zur Messung der Hauttopographie emittiert Licht in einem Wellenlängenbereich zwischen ca. 350 bis 400 nm, wobei der CCD-Chip der Videokamera für den genannten Wellenlängenbereich relativ zu sichtbarem Licht eine höhere Empfindlichkeit aufweist. Die im Vergleich zu sichtbarem Licht kürzere Wellenlänge ermöglicht eine kontrastreiche Oberflächenreflektion, bei der Videobilder mit erheblich erhöhtem Kontrast herstellbar sind.

Die Bildverarbeitungseinrichtung weist eine Bildanalyseeinrichtung auf, die anhand des Online-Videosignals der Videokamera oder anhand der gespeicherten Videosignale die Messung der Hautparameter, die statistische Auswertung und die Dokumentation der Hautparameterdaten durchführt.

Weitere vorteilhafte Merkmale der Erfindung sind den weiteren Unteransprüchen zu entnehmen.

Im folgenden werden unter Bezugnahme auf die Zeichnungen Ausführungsbeispiele der Erfindung näher erläutert.

Es zeigen:
- Fig. 1: eine perspektivische Ansicht des erfindungsgemäßen Applikators mit einer Videokamera und einem in den Applikator integrierten Neon-Lichtrohr,
- Fig. 2: einen Schnitt durch eine Reflexionskappe,
- Fig. 3: eine Frontansicht des Applikators, und
- Fig. 4: einen partiellen Querschnitt durch den Applikator.

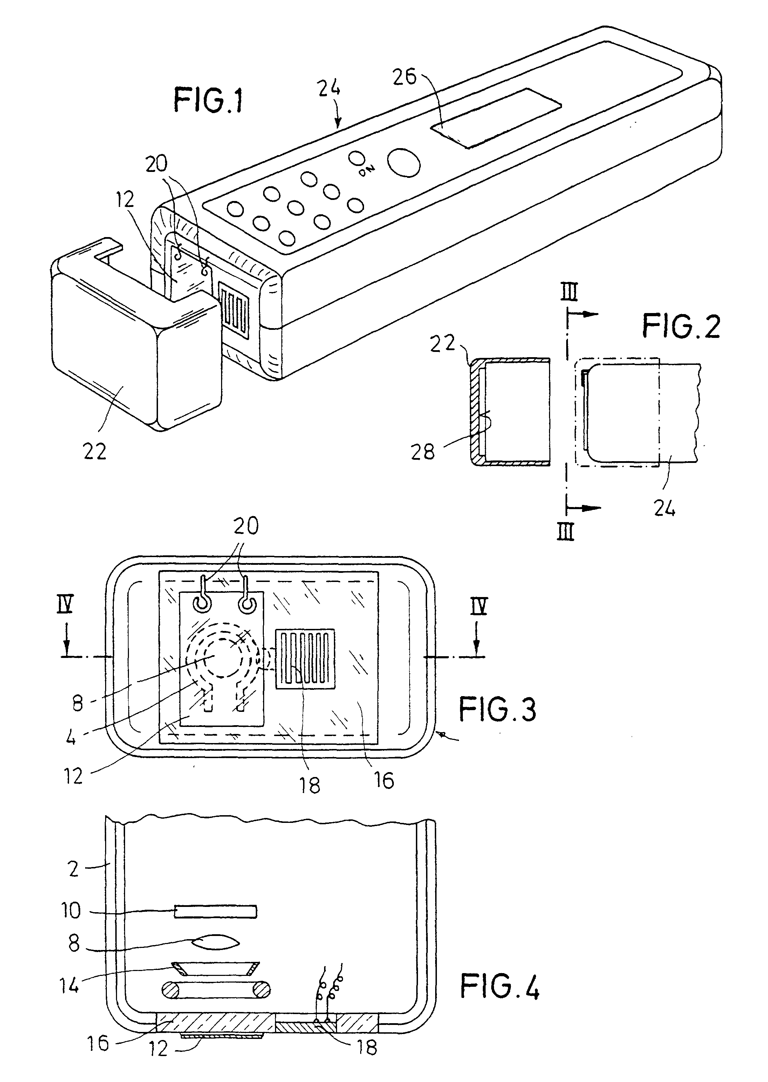

Ein Applikator 2 mit einem Gehäuse, das eine aus einem Neon-Lichtrohr 4 bestehende Lichtquelle, sowie eine aus einer Videokamera bestehende optische Aufnahmeeinrichtung aufnimmt, ist in Fig. 1 gezeigt. Der auf die Hautoberfläche aufsetzbare Applikator 2 hält die Videokamera und die Neonröhre 4 in einem vorbestimmten Abstand von der Hautoberfläche. Die Stromversorgung erfolgt beispielsweise mit Hilfe einer Lithiumbatterie oder einem Akkumulator, die ebenfalls in dem Applikator 2 angeordnet sind.

Durch die feste Vorgabe des Abstandes zur Hautoberfläche kann eine optische Aufnahmeeinrichtung mit fester Fokussierung verwendet werden. Alternativ kann die Optik der Videokamera aus einer variablen Optik bestehen, die unterschiedliche Vergrößerungsfaktoren zuläßt.

Die Neonröhre 4 ist vorzugsweise ringförmig um die optischen Linsen 8 der Videokamera angeordnet.

Anstelle der Neonröhre 4 kann auch eine andere lichtemittierende Einrichtung verwendet werden. Im Falle einer externen Lichtquelle kann das Licht über Lichtleiter in den Applikator 2 geführt werden.

Der Applikator weist vorzugsweise eine eigene Stromversorgung auf, so daß lediglich das Videosignal an eine Bildverarbeitungseinrichtung übertragen werden muß. Hierbei ist es auch möglich, das Videosignal per Funk an die Bildverarbeitungseinrichtung zu übertragen.

Vorzugsweise ist vorgesehen, daß die Neonröhre oder eine sonstige Lichtquelle zur Messung der Hauttopographie Licht in einem Wellenlängenbereich zwischen ca. 350 bis 400 nm emittiert . In diesem Wellenlängenbereich kann die Haut mit einem erhöhten Kontrastumfang von der Videokamera aufgenommen werden, wobei der CCD-Chip 10 der Videokamera für den genannten Wellenbereich relativ zu sichtbarem Licht eine höhere Empfindlichkeit aufweisen kann. Die Erhöhung des Kontrastumfangs durch eine verbesserte Oberflächenreflektion hat den Vorteil, daß die Bildanalyse und Bildverarbeitung mit einer höheren Genauigkeit und besseren Reproduzierbarkeit durchgeführt werden kann. Ein weiterer Vorteil besteht darin, daß abgestorbene Schuppen zu Fluoreszenz angeregt werden und bei der Bildanalyse dadurch besonders hervorgehoben sind.

Der gleiche Applikator 2 kann auch zur Messung der Sebumabsonderung der Hautoberfläche verwendet werden. Hierzu wird eine fettabsorbierende, zunächst undurchsichtige, mit der Fettaufnahme transparent werdende Folie mit Abstand vor der optischen Einrichtung auswechselbar angeordnet, wobei die Folie direkt auf die zu untersuchende Hautoberfläche aufsetzbar ist. Die fettaufnehmende Folie 12 besteht beispielsweise aus einer mikroporösen, hydrophoben Polyprophylen-Folie mit einer Porösität zwischen 20 und 50 %, einer Dicke zwischen 20 und 30 µm und einer Porengröße zwischen 0,03 und 0,15 µm. Eine solche Folie weist eine gleichförmige Mikroporenverteilung auf und ist in ihrem Ursprungszustand opak. Bei Kontaktierung mit Fett füllen sich die Poren, wodurch die Folie an den entsprechenden Stellen transparent wird. Eine solche Folie eignet sich daher für vielfältige Untersuchungen hinsichtlich der Fettausscheidung der Haut, wenn die Folie ggf. unter Aufbringung eines leichten Anpressdrucks in Kontakt mit der Hautoberfläche gebracht wird. In Verbindung mit der Videokamera ist eine zeitliche Dokumentation der Fettaufnahme der Folie möglich. Es sind somit dynamische Fettmessungen möglich, die auch die Bestimmung der Fettproduktionsgeschwindigkeit der Haut zulassen.

Zur Messung der Feuchtigkeitsabsonderung der Hautoberfläche wird eine feuchtigkeitsabsorbierende Folie verwendet, die mit der Feuchtigkeitsaufnahme transparent wird.

Der Applikator ermöglicht somit Direktuntersuchung der Hautoberfläche, und zwar hinsichtlich Abschuppungen und Falten inklusiv der Dokumentation und der Vermessung mit Hilfe einer Bildverarbeitungseinrichtung und einer Bildanalyseeinrichtung. Außerdem kann der Applikator dazu verwendet werden, quantitativen dynamische Messungen von Hautabsonderungen durchzuführen und insbesondere die Produktionsgeschwindigkeit der Hautabsonderungen zu bestimmen.

Fig. 3 zeigt einen partiellen Querschnitt durch den Applikator 2. Die Neonröhre 4 kann vor den optischen Linsen 8 oder seitlich der optischen Linsen angeordnet sein. Zwischen der Neonröhre 4 und der optischen Linse oder der Gruppe von optischen Linsen 8 kann eine geeignete Blende 14, z.B. in Ringform angeordnet sein, die eine Blendung des CCD-Chips 10 durch die Neonröhre 4 verhindert.

Als Auflagefläche für den Applikator 2 gegen die Hautoberfläche ist vorzugsweise eine transparente Scheibe 16 in das Gehäuse 2 integriert, die gleichzeitig als Abstandhalter dient, so daß der Betrieb der Videokamera im Fix-Fokus-Betrieb möglich ist. Die Oberfläche der transparenten Scheibe 16 kann entspiegelt sein. Die transparente Scheibe 16 dient, wie aus Fig. 2 ersichtlich, auch zur Aufnahme der fett- bzw. der feuchtigkeitsaufnehmenden Folie 12.

Die Folie 12 wird beispielsweise durch federnde Klammern 20 auf der transparenten Scheibe 16 gehalten.

Es ist auch möglich, eine Folie 12 auf der transparenten Scheibe 16 festzuklemmen, die einen fettaufnehmenden Bereich und einen feuchtigkeitsaufnehmenden Bereich aufweist, so daß die Messungen gleichzeitig erfolgen können.

Alternativ zu einer feuchtigkeitsaufnehmenden Folie kann ein Feuchtigkeitssensor 18, wie aus Fig. 3 ersichtlich, in der transparenten Scheibe 16 angeordnet sein, mit dem zeitgleich oder im zeitlichen Abstand auch eine Hautfeuchtigkeitsmessung erfolgen kann.

Der Feuchtigkeitssensor 18 ist derart in die Scheibe 16 integriert, daß die Oberfläche des Feuchtigkeitssensors bündig mit der äußeren Oberfläche der Scheibe 16 abschließt.

Ein Vorteil des Applikators 2 besteht auch darin, daß sowohl die Analyse der Hauttopographie, bzw. die Hautfettmessung und die Messung der Hautfeuchtigkeit gleichzeitig und in einer gemeinsamen, von der transparenten Scheibe 16 gebildeten Kontaktfläche erfolgen kann.

Wie aus den Fign. 1 und 2 ersichtlich, kann eine Kappe 22 auf den Applikator 2 aufgesetzt werden, wodurch bei der quantitativen Messung der Hautoberflächenabsonderungen aufgrund der konstanten Reflexionseigenschaften der Kappe 22 eine Nullwertmessung durchführbar ist. Die Kappe 22 kann auf der Innenseite eine schwarze oder eine verspiegelte Oberfläche 28 aufweisen.

Die Bildverarbeitungseinrichtung weist eine Bildanalyseeinrichtung auf, die anhand des Online-Videosignals der Videokamera oder anhand gespeicherter Videosignale die Messung der Hautparameter, die statistische Auswertung und die Dokumentation der Hautparameterdaten durchführt. Dabei kann die Bildanalyseeinrichtung bei Hautabsonderungsmessungen die zeitliche Veränderung der Videosignale zur Bestimmung der Hautabsonderungen-Produktionsgeschwindigkeit der Hautoberfläche auswerten.

In Fig. 1 ist schematisch ein Bedienfeld 24 dargestellt, mit dem die Funktionen des Applikators 2 steuerbar sind.

Desweiteren kann auf dem Applikator 2 ein Anzeigefeld 26, z.B. zur Anzeige eines Meßwertes oder einer Funktion, angeordnet sein.

## Patentansprüche

1. Vorrichtung zur messtechnischen Erfassung von Parametern der Haut von Lebewesen auf einer zu untersuchende Hautoberfläche mit einem Gehäuse, mit einer Lichtquelle, einer optischen Aufnahmeeinrichtung, und mit einer Bildverarbeitungseinrichtung, wobei das Gehäuse aus einem Applikator (2) besteht, in den die aus einer Videokamera bestehende optische Aufnahmeeinrichtung und eine mit der Lichtquelle verbundene lichtemittierende Einrichtung integriert sind, und wobei der auf die Hautoberfläche aufsetzbare Applikator (2) die Videokamera (6) und die Lichtquelle in einem vorbestimmten Abstand von der Hautoberfläche hält,
**dadurch gekennzeichnet,**
**dass** der Applikator (2) eine eine transparente Kontaktfläche bildende Scheibe (16) zum Aufsetzen des Applikators (2) auf die Hautoberfläche aufweist, und
**dass** zur Messung der Absonderung der Haut eine die Absonderungen absorbierende, zunächst undurchsichtige, mit der Aufnahme von Hautabsonderungen transparent werdende Folie (12) auf der Scheibe (16) auswechselbar angeordnet ist, wobei die Folie (12) direkt auf die zu untersuchende Hautoberfläche aufsetzbar ist.

2. Vorrichtung nach Anspruch 1, **dadurch gekennzeichnet, dass** die Folie (12) aus einer fettabsorbierenden, zunächst undurchsichtigen, mit der Fettaufnahme transparent werdenden Folie besteht.

3. Vorrichtung nach Anspruch 1, **dadurch gekennzeichnet, dass** die Folie (12) aus einer feuchtigkeitsabsorbierenden, zunächst undurchsichtigen, mit der Feuchtigkeitsaufnahme transparent werdenden Folie besteht.

4. Vorrichtung nach einem der Ansprüche 1 bis 3, **dadurch gekennzeichnet, dass** die Lichtquelle aus einem Neonlichtrohr (4) besteht, das in den Applikator integriert ist.

5. Vorrichtung nach einem der Ansprüche 1 bis 3, **dadurch gekennzeichnet, dass** die Lichtquelle extern angeordnet ist und über Lichtleiter mit der lichtemittierenden Einrichtung verbunden ist.

6. Vorrichtung nach einem der Ansprüche 1 bis 5, **dadurch gekennzeichnet, dass** die lichtemittierende Einrichtung ringförmig um die Optik (8) der Videokamera angeordnet ist.

7. Vorrichtung nach einem der Ansprüche 1 bis 6, **dadurch gekennzeichnet, dass** das Videosignal der Videokamera per Funk an die Bildverarbeitungseinrichtung übertragbar ist.

8. Vorrichtung nach einem der Ansprüche 1 bis 7, **dadurch gekennzeichnet, dass** die Lichtquelle zur Messung der Hauttopographie Licht in einem Wellenlängenbereich zwischen ca. 350 bis 400 nm emittiert, wobei der CCD-Chip (10) der Videokamera für den genannten Wellenlängenbereich relativ zu sichtbarem Licht eine höhere Empfindlichkeit aufweist.

9. Vorrichtung nach einem der Ansprüche 1 bis 8, **dadurch gekennzeichnet, daß** die Bildverarbeitungseinrichtung den zeitlichen Verlauf der Fett- oder Feuchtigkeitsabsorption in der Folie (12) in Form von Videosignalen speichert.

10. Vorrichtung nach einem der Ansprüche 1 bis 9, **dadurch gekennzeichnet, dass** eine Reflexionskappe (22) auf die Folie (12) zur Bestimmung eines Referenzmesswertes aufsetzbar ist.

11. Vorrichtung nach einem der Ansprüche 1 bis 10, **dadurch gekennzeichnet, dass** die Bildverarbeitungseinrichtung eine Bildanalyseeinrichtung aufweist, die anhand des On-line-Videosignals der Videokamera oder anhand der gespeicherten Videosignale die Messung der Hautparameter, die statistische Auswertung und die Dokumentation der Hautparameterdaten durchführt.

12. Vorrichtung nach Anspruch 11, **dadurch gekennzeichnet, dass** die Bildanalyseeinrichtung die zeitliche Veränderung der Videosignale zur Bestimmung der Fett- oder Feuchtigkeitsproduktionsgeschwindigkeit der Haut auswertet.

13. Vorrichtung nach einem der Ansprüche 1 bis 12, **dadurch gekennzeichnet, dass** eine Stromversorgungseinrichtung in den Applikator (2) integriert ist.

## Claims

1. A device for measuring the skin parameters of living beings on the surface of the skin to be examined, comprising a housing with a light source, an optical recording means, and an image processing means, the housing consisting of an applicator (2), in which an optical recording means formed by a video camera and a light emitting means connected to a light source are integrated, and the applicator (2), which is adapted to be placed on the skin surface, holds the video camera (6) and the light emitting means at a predetermined distance from the skin surface,
**characterized in that**
the applicator (2) comprises a pane (16) forming a transparent contact surface for setting the applicator (2) on the skin surface, and
for measuring the secretions from the skin, a replaceable secretion absorbing film (12) that is initially opaque and becomes transparent with the absorption of skin secretions, is arranged at a distance in front of the optical recording means, the film (12) being adapted to set directly on the skin surface to be examined.

2. The device of claim 1, wherein the film (12) is sebum absorbing film that is initially opaque and becomes transparent as it absorbs sebum.

3. The device of claim 1, wherein the film (12) is a humidity absorbing film that is initially opaque and becomes transparent as it absorbs humidity.

4. The device of one of claims 1 to 3, wherein the light source is a neon light tube (4) integrated in the applicator.

5. The device of one of claims 1 to 3, wherein the light source is external and is connected with the light emitting means via light guides.

6. The device of one of claims 1 to 5, wherein the light emitting means is arranged annularly about the optics (8) of the video camera.

7. The device of one of claims 1 to 6, wherein the video signal from the video camera may be transmitted to the image processing means by radio.

8. The device of one of claims 1 to 7, **characterized in that**, for measuring the topography of the skin, the light source emits light in a wavelength range between about 350 and 400 nm, the CCD chip (10) of the video camera being more sensitive to this wavelength range than to visible light.

9. The device of one of claims 1 to 8, wherein the image processing means stores the variation in time of the absorption of sebum or humidity in the film (12) as a video signal.

10. The device of one of claims 1 to 9, wherein a reflection cap (22) may be placed on the film (12) to determine a reference measuring value.

11. The device of one of claims 1 to 10, wherein the image processing means comprises an image analyzing means for performing the measuring of the skin parameters, the statistic evaluation and the documentation of the skin parameter data using the online video signal of the video camera or the stored video signals.

12. The device of claim 11, wherein the image analyzing means evaluates the variation in time of the video signals to determine the rate at which the skin produces sebum and humidity.

13. The device of one of claims 1 to 12, wherein a current supply means is integrated in the applicator (2).

## Revendications

1. Dispositif de mesure des paramètres de la peau d'êtres vivants, sur une surface de peau à analyser, comportant un boîtier, une source de lumière, un dispositif de réception optique, et un dispositif de traitement de l'image, le boîtier se composant d'un applicateur (2) dans lequel sont intégrés le dispositif de réception optique, consistant en une caméra vidéo, et un dispositif émettant de la lumière relié à la source de lumière, et l'applicateur (2), qui peut être posé sur la surface de la peau, maintenant la caméra vidéo (6) et la source de lumière à une distance prédéterminée de la surface de peau, **caractérisé en ce que**,
l'applicateur (2) comporte une plaque (16) formant une surface de contact transparente pour poser l'applicateur (2) sur la surface de la peau, et
**en ce que**, pour la mesure de la sécrétion de la peau, une feuille (12) absorbant les sécrétions, à l'origine opaque, devenant transparente par l'absorption des sécrétions de la peau, est placée sur la plaque (16) de manière interchangeable, la feuille (12) pouvant être placée directement en contact avec la surface de peau à examiner.

2. Dispositif selon la revendication 1, **caractérisé en ce que** la feuille (12) se compose d'une feuille absorbant les matières grasses, à l'origine opaque, devenant transparente avec l'absorption des matières grasses.

3. Dispositif selon la revendication 1, **caractérisé en ce que** la feuille (12) se compose d'une feuille absorbant l'humidité, à l'origine opaque, devenant transparente avec l'absorption de l'humidité.

4. Dispositif selon l'une des revendications 1 à 3, **caractérisé en ce que** la source de lumière consiste en un tube lumineux au néon (4) qui est intégré dans l'applicateur.

5. Dispositif selon l'une des revendications 1 à 3, **caractérisé en ce que** la source de lumière est disposée à l'extérieur et est reliée au dispositif émetteur de lumière par un guide de lumière.

6. Dispositif selon l'une des revendications 1 à 5, **caractérisé en ce que** le dispositif émetteur de lumière est disposé en forme d'anneau autour de l'optique (8) de la caméra vidéo.

7. Dispositif selon l'une des revendications 1 à 6, **caractérisé en ce que** le signal vidéo de la caméra vidéo est transmissible par radio au dispositif de traitement de l'image.

8. Dispositif selon l'une des revendications 1 à 7, **caractérisé en ce que** la source de lumière, pour la mesure de la topographie de la peau, émet de la lumière dans un domaine de longueurs d'onde entre environ 350 et 400 nm, la puce CCD (10) de la caméra vidéo présentant une sensibilité plus élevée pour ledit domaine de longueurs d'onde relativement à la lumière visible.

9. Dispositif selon l'une des revendications 1 à 8, **caractérisé en ce que** le dispositif de traitement de l'image mémorise sous forme de signaux vidéo l'évolution dans le temps de l'absorption de matière grasse ou d'humidité dans la feuille (12).

10. Dispositif selon l'une des revendications 1 à 9, **caractérisé en ce qu'**un capuchon réfléchissant (22) est apte à être mis en place sur la feuille (12) pour la détermination d'une valeur de mesure de référence.

11. Dispositif selon l'une des revendications 1 à 10, **caractérisé en ce que** le dispositif de traitement de l'image comporte un dispositif d'analyse de l'image qui, à l'aide du signal vidéo en ligne de la caméra vidéo ou à l'aide des signaux vidéo mémorisés, exécute la mesure des paramètres de la peau, l'exploitation statistique et la documentation des données des paramètres de peau.

12. Dispositif selon la revendication 11, **caractérisé en ce que** le dispositif d'analyse de l'image exploite le changement dans le temps des signaux vidéo pour la détermination de la vitesse de production de matière grasse ou d'humidité de la peau.

13. Dispositif selon l'une des revendications 1 à 12, **caractérisé en ce qu'**un dispositif d'alimentation en courant électrique est intégré dans l'applicateur (2).
